# EUROPÄISCHE PATENTSCHRIFT

(11) **EP 1 932 483 B1**
(45) Veröffentlichungstag und Bekanntmachung des Hinweises auf die Patenterteilung: **13.02.2013**
(21) Anmeldenummer: 07021844.1
(22) Anmeldetag: 09.11.2007
(51) Int. Cl.: A61B 17/00

(54) **Medizinische Einrichtung zur Lagebestimmung von intrakorporalen Implantaten**
Medical device for determining the position of intracorporal implants
Dispositif médical destiné à la détermination spatiale d'implants intracorporels

(30) Priorität: 13.12.2006 DE 102006059225
(43) Veröffentlichungstag der Anmeldung: 18.06.2008
(73) Patentinhaber: Wittenstein AG, 97999 Igersheim (DE)
(72) Erfinder: Wrobel, Miroslaw, 97753 Karlstadt (DE); Stauch, Roman, 97959 Assamstadt (DE)
(74) Vertreter: Weiss, Peter

(56) Entgegenhaltungen:
- WO-A-98/19619
- US-A1- 2003 078 495

## Beschreibung

Die Erfindung betrifft eine medizinische Einrichtung nach den Merkmalen des Oberbegriffs des Anspruchs 1.

Bei der medizinischen Behandlung von z. B. Wirbelfrakturen, die durch Stürze, Osteoporose, Tumore etc. verursacht sein können, werden vielfach Fixationssysteme eingesetzt, bei denen eine Art von Ösenschrauben, genannt Pedikelschrauben zum Einsatz kommen, durch deren jeweilige Ösen die Fixationsstäbe gesteckt werden.

Nach der konventionellen Operationsmethode werden zur Einbringung der Pedikelschrauben und Fixationsstäbe die Muskeln großflächig von der Wirbelsäule abgelöst, wobei von den Pedikelschrauben jeweils eine zu beiden Seiten des Rückenmarkkanals eines Wirbelkörpers der Wirbelsäule in diesen geschraubt und die Fixationsstäbe von oben nach unten durch die Ösen der Pedikelschrauben gesteckt werden.

Bei jungen Menschen werden die Fixationsstäbe nach ca. einem ¾ Jahr entfernt, da sonst die Gefahr besteht, dass die Schrauben/Stäbe mit zunehmender Mobilität des Patienten brechen könnten.

Bei älteren Menschen mit z. B. osteoporotischen Schäden (Frakturen, Sinterungen) an der Wirbelsäule wird vielfach bei einer Behandlung mit einem Stabsystem (Fixateur interne, ggf. in Verbindung mit Knochenzement) oder einem Plattensystem nach dem Fixateurprinzip in der Regel verbleibend fusioniert. Jedoch besteht hierbei die Gefahr eines "Durchschneidens" der Schrauben in den Bandscheibenraum wegen des verminderten Knochenwiderstands bei Osteoporose.

Demgegenüber erfolgt bei der sogenannten "Minimalinvasiven Methode" kein Freilegen der Wirbelsäule, sondern die Behandlung erfolgt lediglich durch kleine Schnitte von außen durch die Haut. Unter diese Methode fällt z. B. die Vertebroplastie, bei der Knochenzement ohne vorausgehende Wiederaufrichtung der Wirbelkörper in diese gespritzt wird, oder die Kyphoplastie, bei der eingebrochene Wirbel mit Hilfe eines aufblasbaren Ballons und sodann eingespritztem Biozement wieder aufgerichtet werden. Dagegen ist das Einbringen von Fixationsstäben in der Frakturbehandlung von Wirbelkörpern durch ein minimalinvasives Verfahren bislang nicht möglich gewesen.

So zeigen beispielsweise die US 2003/0078494 A1 und die US 2006/0241401 A1 Verfahren mit denen Herzkatheter mittels Impendanz positioniert bzw. auf ihre Position und Funktion überprüft werden können. Dazu wird in die Nähe des Herzkatheters ein Referenzteil in den Körper eingebracht und dessen Position absolut ermittelt. Der Abstand zwischen dem Referenzteil und dem Herzkatheter kann per Impendanz, Ultraschall oder eine Kombination der beiden Verfahren ermittelt werden.

In diesem Zusammenhang ist es aus der US2003/0078495 A1 bekannt, die Lage von Implantaten mittels Ultraschall zu bestimmen.

Hier setzt die Erfindung ein, der die Aufgabe zugrunde liegt, eine Möglichkeit zu schaffen, die Lage von intrakorporalen Implantaten in einfacher Weise bestimmen zu können, ohne dass dafür größere Operationseingriffe erforderlich sind.

Diese Aufgabe wird erfindungsgemäß durch die Merkmale des kennzeichnenden Teils des Anspruchs 1 gelöst. Es wird eine medizinische Einrichtung verwendet, die eine Impedanz-Messeinrichtung und eine Ultraschall-Einrichtung umfasst, die beide mit mindestens einer intrakorporale Sonde in Verbindung stehen, wobei die Impedanz-Messeinrichtung zusätzlich noch mit mindestens einem Anschluss für eine elektrisch leitende Verbindung mit dem Implantat ausgestattet ist.

Nach Maßgabe der Erfindung dient bei der Impedanzmessung als Messgröße zur Lagebestimmung die Phasenverschiebung zwischen Strom und Spannung, die in Abhängigkeit zum Abstand der intrakorporalen Sonde und dem Implantat zueinander auftritt, d. h., ist diese klein, dann bedeutet dies geringer Abstand und ist diese groß, dann bedeutet dies großer Abstand. Daraus ergibt sich: Die Größe der Impedanz ist eine Funktion des Abstands zwischen Sonde und Implantat.

Die Kombination von Impedanzmessung und Ultraschall hat den Vorteil, dass die Impedanz-Messeinrichtung für eine grobe und die Ultraschall-Einrichtung für eine genaue Lagebestimmung eingesetzt werden kann, wobei unter grobe Lagebestimmung die erste Annäherung der Sonde an das Implantat zu verstehen ist, wogegen dann der Restweg der Sonde bis zu dem oder sogar in das Implantat durch die genaue Lagebestimmung mit Hilfe der Ultraschall-Einrichtung erfolgt.

Mit anderen Worten im Vergleich mit der Beleuchtung eines Kraftfahrzeuges: "Fernlicht" für die grobe Positionierung durch Annäherung über die Impedanzmessung, wobei die Gewebezusammensetzung hierbei auch messbar ist, d. h., welches Gewebe gerade passiert wird und welche Vitalität dieses aufweist. Sobald dann hierbei eine relative Annäherung an das Implantat erreicht ist, wird auf "Abblendlicht" bzw. Ultraschall zur genaueren Positionierung umgeschaltet.

Falls mehrere Implantate z. B. hintereinander vorhanden sind, ist es zweckmäßig, die Impedanz-Messeinrichtung und die Ultraschall-Einrichtung nacheinander schaltbar auszulegen, d. h. die grobe und die genaue Lagebestimmung erfolgt je fortschreitendem Implantat von neuem.

Ferner ist es möglich, die Impedanz-Messeinrichtung und die Ultraschall-Einrichtung derart zu schalten, dass sie gleichzeitig aktiv sind, was die Sondenführung verbessert.

Der wesentliche Vorteil der erfindungsgemäßen medizinischen Einrichtung liegt in der Verwendung bei der minimalinvasiven Chirurgie, denn dadurch müssen z. B. ausgedehnte Gewebe- und/oder Muskelbereiche nicht mehr abgelöst werden, wodurch eine Verminderung des Blutverlustes, eine geringere Muskelnervenschädigung, die Erhaltung der Propriorezeption, eine geringere Narbenbildung an Haut - und Muskelbereich und damit eine Reduzierung der Liegezeiten sowie eine frühere Mobilisierung der Patienten möglich ist. Schließlich kann auch die Röntgen-Strahlenbelastung durch den Einsatz der Ultraschalltechnologie für den Arzt und den Patienten während der intraoparativen Applikation reduziert werden.

Die erfindungsgemäße Einrichtung ist insbesondere zur Lagebestimmung bei einem Wirbelsäulenfixationssystem mit Stäben als Stabilisatoren und mit sogenannten Pedikelschrauben in der minimalinvasiven Chirurgie einsetzbar, und zwar dienen hier die Stäbe als intrakorporale Sonden mit denen die Impedanz-Messeinrichtung und die Ultraschall-Einrichtung in Verbindung stehen, wobei der zusätzliche Anschluss der Impedanz-Messeinrichtung mit den Pedikelschrauben in elektrisch leitender Verbindung steht.

Es ist weiter erfindungsgemäß vorgesehen, dass die intrakorporale Sonde stabförmig ausgebildet ist und an ihrem freien Handende im wesentlichen eine Ultraschall-Einrichtung und einen Messwandler aufweist, wobei deren Befestigung an der Sonde durch ein Gewinde, einen Bajonettverschluss oder einen Klemmverschluss erfolgt, damit am Ende einer Operation wieder eine Entfernung erfolgen kann. Gleichzeitig muss aber sichergestellt sein, dass sich diese Gewindeverbindung beim Manipulieren während einer Operation nicht lösen kann, was insbesondere nicht bei einer Drehung der Sonde nach links und nach rechts gegeben sein darf, weswegen hier ein geeignetes Feingewinde zu bevorzugen ist. Ferner sind Eindrehungen im Stab zur Fokussierung des Ultraschalls notwendig, wobei sich diese auch im Handstück befinden können.

Das Arbeiten mit der erfindungsgemäßen Einrichtung erfolgt in der Weise, dass zuerst mit der Sonde bei deren intrakorporaler Einführung eine grobe Lagebestimmung durchgeführt wird, und zwar indem mit Hilfe der Impedanz-Messeinrichtung die Phasenverschiebung zwischen Strom und Spannung in Abhängigkeit zum Abstand der Sonde und dem Implantat zueinander gemessen wird, während kurz vor dem Erreichen des Implantats durch die Sonde eine genaue Lagebestimmung erfolgt, die dann mit Hilfe der Ultraschall-Einrichtung durchgeführt wird.

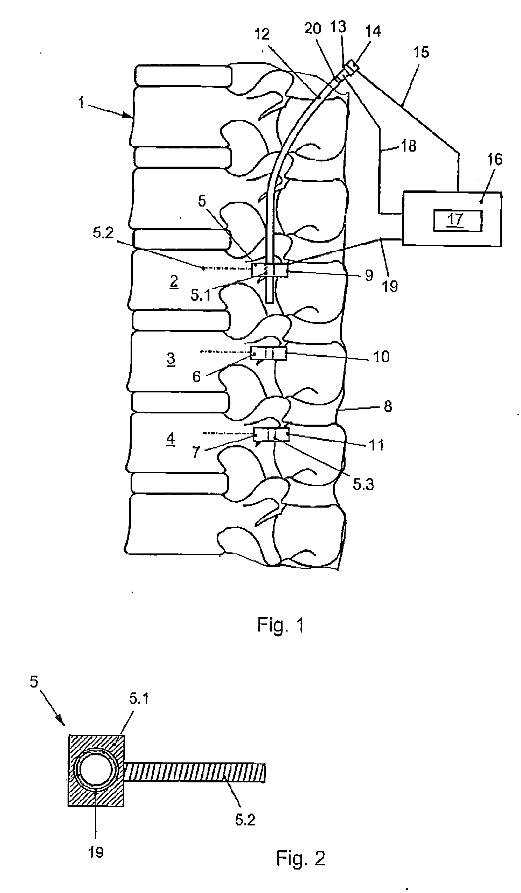

Weitere Merkmale und Vorteile der vorliegenden Erfindung ergeben sich aus der nachfolgenden Beschreibung eines Ausführungsbeispiels in Verbindung mit der Zeichnung, welche lediglich eine Fig.1 zeigt, in der schematisch die Seitenansicht eines Abschnitts einer Wirbelsäule zu sehen ist, an der erfindungsgemäß drei Wirbelkörper miteinander verbunden, d. h. stabilisiert werden sollen.

Im Einzelnen zeigt die Fig. 1 einen seitlichen Abschnitt einer Wirbelsäule 1, in deren drei Wirbelkörper 2 - 4 jeweils so genannte Pedikelschrauben 5 - 7 geschraubt sind, wozu die mit 8 bezeichnete Haut lediglich relativ kleine Schnitte erhält, - angedeutet bei 9 -11- . Die einzelnen Pedikelschrauben 5 - 7 bestehen im wesentlichen - am Beispiel der ersten Schraube 5 - aus einer Öse 5.1 und einem Gewindeschaft 5.2 und sind aus Titan gefertigt, wobei Chrom-, Kobalt-Nickellegierungen ebenfalls möglich sind.

Die drei Wirbelkörper 2 - 4 sollen miteinander verbunden, d. h. stabilisiert werden, wozu nach dem minimalinvasiven Verfahren ein Fixationsstab 12 dient, der am seinem Kopfende einen Ultraschall-Einrichtung 13 und einen Messwertumwandler 14 besitzt, der über eine elektrische Leitung 15 mit einer Impedanz-Messeinrichtung 16 mit einer optischen und/oder akustischen Anzeigeeinrichtung 17 verbunden ist. Gleichzeitig ist die Impedanz-Messeinrichtung 16 über die Leitung 18 mit dem Fixationsstab 12 verbunden und über einen Anschluss 19 mit der Öse 5.1 der Pedikelschraube 5.

Das minimalinvasive Setzen der Pedikelschrauben 5 - 7 und des Fixationsstabes 12 geschieht wie folgt, wobei in Fig. 1 nur eine Hälfte der Schraubenanordnung gezeigt ist, denn praktisch werden zu beiden Seiten des Rückenmarkkanals der betroffenen Wirbelkörper 2 - 4 eine Schraube gesetzt, sodass zwei Reihen Schrauben mit je einem Fixationsstab 12 vorhanden sind, von denen jedoch nur eine Reihe in Fig. 1 zu sehen ist.

Zum Eindrehen der Pedikelschrauben 5 - 7 werden in der Haut 8 entsprechend kurze Schnitte 9 - 11 eingebracht und die Pedikelschrauben 5 - 7 nach einem Vorbohren mit ihrem jeweiligen Gewindeschaft (Beispiel: 5 → 5.2) in die Wirbelkörper 2 - 4 (Beispiel: 2) derart eingeschraubt, dass ihre Ösen (Beispiel: 5.1) waagrecht stehen. Hierbei ist zu beachten, dass die Schrauben 5 - 7 durch den jeweiligen Muskel hindurch gesetzt werden, d. h. hierzu soll nach Möglichkeit eine Längsteilung bzw. Verdrängung des Muskels erfolgen.

Zum minimalinvasiven Einbringen des Fixationsstabes 12 durch die Ösen der Pedikelschrauben 5 - 7, soll der Stab ebenfalls nach Möglichkeit durch den Muskel geschoben werden, wobei zu Beginn die Haut 8 durch einen nicht näher bezeichneten Schnitt durchdrungen werden muss.

Als Zielhilfe für den Fixationsstab 12 dient zu Beginn die Impedanzmessung, bei der als Messgröße die Phasenverschiebung zwischen Strom und Spannung dient, welche über eine Anzeigeeinrichtung 17, insbesondere optischer und/oder akustischer Art ablesbar ist, d. h. je kleiner diese Phasenverschiebung ist, desto näher befindet sich die Spitze des Fixationsstabes 12 an der Öse 5.1. Wenn dieses "Δ klein" einen bestimmten Nahbereich erreicht hat, wird auf den Ultraschall-Einrichtung 13 umgeschaltet, da mit diesem der Fixationsstab 12 genauer und somit besser durch die Öse 5.1 geführt werden kann.

Das Weiterführen des Fixationssabes 12 durch die Ösen der beiden weiteren Pedikelschrauben 6 und 7 erfolgt analog, wobei dann die Leitung 19 jeweils mit den Ösen dieser Schrauben verbunden werden muss. Hierbei können zwei Möglichkeiten gewählt werden, und zwar erst die Impedanzmethode als Groborientierung und dann für die genauere Annäherung und das Durchführen durch die Öse der Ultraschall oder beides .

Die Befestigung des Ultraschall-Einrichtung 13 mit dem Messwandler 14 an dem Fixationsstab 12 als Sonde ist durch ein Feingewinde, Bajonettverschluss od. dgl. bewerkstelligt, und zwar damit diese Einheit am Ende des Eingriffs wieder entfernt werden kann.

Ferner besitzt der in der Wirbelsäule 1 verbleibende Abschnitt des Fixationsstabes 12 an seinem Außenumfang eine Kerbe 20, damit dieser Abschnitt zu gegebener Zeit mit Hilfe eines geeigneten Extraktionsinstruments wieder entfernt werden kann.

### Bezugszeichenliste

| | | | | | |
|---|---|---|---|---|---|
| 1 | Wirbelsäule | 34 | | 67 | |
| 2 | Wirbelkörper | 35 | | 68 | |
| 3 | Wirbelkörper | 36 | | 69 | |
| 4 | Wirbelkörpe | 37 | | 70 | |
| 5 | Pedikelschraube | 38 | | 71 | |
| 5.1 | Öse | | | | |
| 5,2 | Gewindeschaft | | | | |
| 6 | Pedikelschraube | 39 | | 72 | |
| 7 | Pedikelschraube | 40 | | 73 | |
| 8 | Haut | 41 | | 74 | |
| 9 | Schnitt | 42 | | 75 | |
| 10 | Schnitt | 43 | | 76 | |
| 11 | Schnitt | 44 | | 77 | |
| 12 | Sonde/Fixationsstab | 45 | | 78 | |
| 13 | Ultraschallkopf | 46 | | 79 | |
| 14 | Messwertwandler | 47 | | | |
| 15 | Leitung | 48 | | | |
| 16 | Impedanz-Messeinrichtung | 49 | | | |
| 17 | Anzeigeeinrichtung | 50 | | | |
| 18 | Leitung | 51 | | | |
| 19 | Anschluss | 52 | | | |
| 20 | Kerbe | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Medizinische Einrichtung zur Lagebestimmung von intrakorporalen Implantaten
umfassend
- mindestens eine Ultraschall-Einrichtung (13),
- eine Impedanz-Messeinrichtung (10)
- eine intrakorporale Sonde (12), wobei sie stabförmig ausgebildet ist und an ihrem freien Handende im Wesentlichen die Ultraschall-Einrichtung (13) und einen Messwandler (14) aufweist,
- ein Implantat (5), wobei die Ultraschall-Einrichtung (13) und die Impedanz-Messeinrichtung (16) mit der intrakorporalen Sonde (12) in Verbindung stehen, wobei die Impedanz-Messeinrichtung (16) mit mindestens einem Anschluss (19) ausgestattet ist, wobei der Anschluss (19) über eine elektrisch leitende Verbindung mit dem Implantat (5) verbindbar ist,
**dadurch gekennzeichnet,**
**dass** die Befestigung der Ultraschall-Einrichtung (13) mit dem Messwandler (14) an der Sonde (12) durch ein Gewinde, insbesondere eine Feingewinde, einen Bajonettverschluss oder einen Klemmverschluss erfolgt.

2. Medizinische Einrichtung nach Anspruch 1, **dadurch gekennzeichnet, dass** zur Lagebestimmung bei der Impedanzmessung als Messgröße die Phasenverschiebung zwischen Strom und Spannung dient, die in Abhängigkeit zum Abstand der intrakorporalen Sonde (12) und dem Implantat (5) zueinander auftritt.

3. Medizinische Einrichtung nach einem der Ansprüche 1 oder 2, **dadurch gekennzeichnet, dass** die Impedanz-Messeinrichtung (16) für eine grobe und die Ultraschall-Einrichtung (13) für eine genaue Lagebestimmung dienen.

4. Medizinische Einrichtung nach Anspruch 3, **dadurch gekennzeichnet, dass** die Impedanz- Messeinrichtung (16) und die Ultraschall-Einrichtung (13) alternierend schaltbar sind.

5. Medizinische Einrichtung nach einem der Ansprüche 1 bis 3, **dadurch gekennzeichnet, dass** die Impedanz-Messeinrichtung (16) und die Ultraschall-Einrichtung (13) derart geschaltet sind, dass sie gleichzeitig aktiv sind.

6. Medizinische Einrichtung nach einem der Ansprüche 1 bis 5, **dadurch gekennzeichnet, dass** der an einer Wirbelsäule (1) verbleibende Abschnitt der stabförmigen Sonde (12) zumindest an einem seiner Enden eine Vertiefung wie Kerbe (20) für das Ansetzen eines Extraktionsinstrumentes aufweist.

7. Medizinische Einrichtung nach Anspruch 1 zur Lagebestimmung von Pedikelschrauben und dgl. bei einem Wirbelsäulenfixationssystem mit stabförmigen Sonden als Stabilisatoren, **dadurch gekennzeichnet, dass** jeweils eine intrakorporale Sonde (12) mit einer Impedanz-Messeinrichtung (16) und einer Ultraschall-Einrichtung (13) in Verbindung steht, wobei ein zusätzlicher Anschluss (19) der Impedanz-Messeinrichtung (16) mit den jeweiligen Pedikelschrauben (5-7) in elektrisch leitender Verbindung steht.

## Claims

1. A medical means for determining the position of intracorporeal implants, comprising
- at least one ultrasound means (13),
- an impedance-measuring means (10),
- an intracorporeal probe (12), it being rod-shaped and having at its free hand end substantially the ultrasound means (13) and a measuring transducer (14),
- an implant (5), the ultrasound means (13) and the impedance-measuring means (16) being connected to the intracorporeal probe (12), the impedance-measuring means (16) being equipped with at least one connector (19), the connector (19) being able to be connected to the implant (5) via an electrically conductive connection,
**characterised in that** the ultrasound means (13) with the measuring transducer (14) is fastened to the probe (12) by a thread, in particular a fine thread, a bayonet lock or a clamping lock.

2. A medical means according to Claim 1, **characterised in that** for determining the position upon the impendance measurement the phase shift between current and voltage which occurs dependent on the distance between the intracorporeal probe (12) and the implant (5) relative to one another serves as the measured variable.

3. A medical means according to one of Claims 1 or 2, **characterised in that** the impedance-measuring means (16) serves for rough determination of position and the ultrasound means (13) for accurate determination of position.

4. A medical means according to Claim 3, **characterised in that** the impedance-measuring means (16) and the ultrasound means (13) can be switched in alternation.

5. A medical means according to one of Claims 1 to 3, **characterised in that** the impedance-measuring means (16) and the ultrasound means (13) are switched such that they are active simultaneously.

6. A medical means according to one of Claims 1 to 5, **characterised in that** the section of the rod-shaped probe (12) which remains on a spinal column (1) has at least at one of its ends a recess such as a notch (20) for applying an extraction instrument.

7. A medical means according to Claim 1 for determining the position of pedicle screws and the like in a spinal fixation system with rod-shaped probes as stabilisers, **characterised in that** in each case an intracorporeal probe (12) is connected to an impedance-measuring means (16) and an ultrasound means (13), an additional connector (19) of the impedance-measuring means (16) being connected in electrically conductive manner to the respective pedicle screws (5-7).

## Revendications

1. Dispositif médical destiné à la détermination spatiale d'implants intracorporels, comprenant,
- au moins un dispositif ultrasonore (13),
- un dispositif de mesure d'impédance (10)
- une sonde intracorporelle (12), cette dernière étant réalisée en forme de barre et présentant à son extrémité manuelle libre substantiellement le dispositif ultrasonore (13) et un convertisseur de mesure (14),
- un implant (5), le dispositif ultrasonore (13) et le dispositif de mesure d'impédance (16) étant en communication avec la sonde intracorporelle (12), le dispositif de mesure d'impédance (16) étant équipé d'une connexion (19), la connexion (19) pouvant être connectée à l'implant (5) par l'intermédiaire d'une connexion électro-conductrice,
**caractérisé par le fait**
**que** la fixation du dispositif ultrasonore (13) avec le convertisseur de mesure (14) à la sonde (12) a lieu par un filetage, en particulier en filetage fin, une fermeture à baïonnette ou une fermeture à pression.

2. Dispositif médical selon la revendication 1, **caractérisé par le fait que** pour la détermination spatiale lors de la mesure d'impédance sert comme grandeur de mesure le déphasage entre le courent et la tension qui se produit en fonction de la distance entre eux de la sonde intracorporelle (12) et de l'implant (5).

3. Dispositif médical selon l'une des revendications 1 ou 2, **caractérisé par le fait que** le dispositif de mesure d'impédance (16) sert à une détermination spatiale approximative et le dispositif ultrasonore (13) sert à une détermination spatiale précise.

4. Dispositif médical selon la revendication 3, **caractérisé par le fait que** le dispositif de mesure d'impédance (16) et le dispositif ultrasonore (13) sont commutables en alternance.

5. Dispositif médical selon l'une des revendications 1 ou 2, **caractérisé par le fait que** le dispositif de mesure d'impédance (16) et le dispositif ultrasonore (13) sont commutés de sorte qu'ils soient actifs en même temps.

6. Dispositif médical selon l'une des revendications 1 ou 5, **caractérisé par le fait que** le segment de la sonde en forme de barre (12) restant sur une colonne vertébrale (1) présente, au moins à l'une de ses extrémités, un creux, tel qu'une encoche (20), pour le placement d'un instrument d'extraction.

7. Dispositif médical selon la revendication 1 destiné à la détermination spatiale de vis pédiculaires et autres dans un système de fixation de colonne vertébrale par des sondes en forme de barres comme stabilisateurs, **caractérisé par le fait que** chaque fois une sonde intracorporelle (12) est en communication avec un dispositif de mesure d'impédance (16) et un dispositif ultrasonore (13), une connexion additionnelle (19) du dispositif de mesure (16) étant en connexion électro-conductrice avec les vis pédiculaires (5 à 7).
